# FASCICULE DE BREVET EUROPEEN

(11) **EP 1 411 115 B1**
(45) Date de publication et mention de la délivrance du brevet: **09.05.2007**
(21) Numéro de dépôt: 03292374.0
(22) Date de dépôt: 26.09.2003
(51) Int. Cl.: C12N 5/00

(54) **Composition pour culture de cellules comprenant du polyéthylène glycol**
Zusammensetzung zur Zellkultur, die Polyethylenglykol enthält
Cell culture composition comprising polyethylene glycol

(30) Priorité: 16.10.2002 FR 0212878
(43) Date de publication de la demande: 21.04.2004
(73) Titulaire: MACO PHARMA, 59420 Mouvaux (FR)
(72) Inventeur: Heron, Antoine, 59250 Halluin (FR)
(74) Mandataire: Geismar, Thierry

(56) Documents cités:
- EP-A- 0 248 656
- WO-A-88/00967
- WO-A-98/30679
- KIPP D E & SCHWARZ R I: "Effectiveness of isoascorbate versus ascorbate as an inducer of collagen synthesis in primary avian tendon cells" JOURNAL OF NUTRITION, vol. 120, no. 2, 1990, pages 185-189, XP008020039 ISSN: 0022-3166

## Description

L'invention concerne un milieu pour culture de cellules notamment animales ou de tissus.

Dans le domaine des milieux de culture in vitro de cellules directement implantables chez l'homme, on connaît déjà des milieux dont le constituant principal est du sérum. Ces milieux présentent toutefois des inconvénients.

Tout d'abord, la composition de tels milieux n'est que partiellement connue, ce qui pose des problèmes notamment en termes de reproductibilité fonctionnelle entre différents sérums, et entre différents lots d'un même sérum.

En outre, le sérum est un vecteur potentiel d'agents pathogènes. En particulier, lorsque le sérum est d'origine bovine, le risque de contamination par les prions n'est pas nul.

Pour remédier à ces inconvénients, il a été proposé des milieux de culture sans sérum, ou « serum-free ». Ces milieux ont pour constituants essentiels des protéines naturelles purifiées faisant office de substituts du sérum, telles que l'albumine, la transferrine et l'insuline. Toutefois, la standardisation de tels milieux demeure difficile à obtenir, et les risques de contamination par des agents pathogènes ne sont pas négligeables.

Par ailleurs, les milieux de culture contenant de telles protéines présentent un coût de production important.

Pour tenter de résoudre ces inconvénients, on connaît, notamment du document WO-98/30679, un milieu de culture comprenant un substitut d'albumine, un substitut de transferrine et un substitut d'insuline. Toutefois, de tels milieux ne sont pas exempts de protéines, en ce que notamment les substituts d'albumine utilisés sont eux-mêmes des protéines.

La demanderesse a donc effectué des essais intensifs pour tenter de substituer l'albumine avec un composant présentant des propriétés analogues, mais sans présenter les inconvénients mentionnés ci-dessus.

La demanderesse a ainsi défini une composition et un milieu de culture comprenant, en tant que substitut de l'albumine, du polyéthylène glycol dans une plage de concentration déterminée, de sorte à assurer les fonctions requises.

Le coût d'une telle composition et d'un tel milieu est nettement inférieur à celui d'une composition ou d'un milieu contenant des protéines naturelles purifiées.

A cet effet, l'invention propose un milieu pour culture de cellules notamment animales ou de tissus, du type comprenant de l'albumine, un substitut de transferrine naturelle, un substitut d'insuline naturelle et du polyéthylène glycol en quantité supérieure ou égale à 1% en poids.

Selon une réalisation, la composition comprend en outre de l'érythorbate de sodium.

D'autres objets et avantages de l'invention apparaîtront au cours de la description qui suit.

Dans cette composition, les protéines naturelles dont le rôle est essentiel, à savoir la transferrine et l'insuline, ont été remplacées par des composants de propriétés équivalentes, mais n'induisant pas de problème de reproductibilité ni de contamination éventuelle par des agents pathogènes.

A cet effet, la transferrine est remplacée de façon connue par un chélateur du fer tel que l'EDTA, l'EGTA ou l'acide gluconique, de sorte à éviter la phase de pasteurisation rendue obligatoire lors de l'utilisation de transferrine humaine purifiée.

L'insuline est par exemple remplacée de façon connue par de l'insuline humaine recombinante, ou par un sel de zinc.

Le polyéthylène glycol présente l'avantage de posséder les mêmes propriétés fonctionnelles que l'albumine dans une culture cellulaire. En effet, au sein d'un milieu de culture, le polyéthylène glycol exerce notamment un effet osmotique, un effet stabilisateur des membranes cellulaires et un effet sur le maintien de la viabilité cellulaire au cours de la culture. En outre, il joue le rôle de détoxifiant et de piégeur de radicaux libres, de sorte à prévenir le cas échéant la péroxydation des lipides membranaires.

Pour exercer de façon optimale le rôle de l'albumine, la quantité de polyéthylène glycol introduite dans la composition est, selon l'invention, supérieure ou égale à 1% en poids.

En particulier, cette quantité est comprise entre 1 et 5% en poids, et notamment entre 1 et 3% en poids.

Le polyéthylène glycol utilisé est par exemple de poids moléculaire compris entre 50 et 100 000 daltons, notamment 20 000 daltons. L'utilisation d'un tel polyéthylène glycol présente l'avantage de répondre pleinement aux exigences de qualité et de sécurité nécessaires pour un usage thérapeutique. En effet, ces polymères sont couramment utilisés en tant que solvants, intermédiaires de synthèse ou excipients pour des préparations cosmétiques et pharmaceutiques et possèdent de la sorte un grade pharmaceutique.

Selon une réalisation, la composition selon l'invention comprend de l'érythorbate de sodium, dans une quantité inférieure ou égale à 0,1 % en poids, notamment comprise entre 10⁻⁶ % et 0,1 % en poids. Plus particulièrement, la quantité d'érythorbate de sodium est inférieure à 5.10⁻³ % en poids, et est notamment comprise entre 10⁻⁴ % et 5.10⁻³ % en poids. L'introduction d'une telle molécule dans la composition est destinée à jouer le rôle de substitut des molécules anti-oxydantes. En effet, l'érythorbate de sodium possède un pouvoir anti-oxydant important, sans pour autant présenter les inconvénients liés par exemple à l'utilisation des vitamines C et E, dont l'effet anti-oxydant est limité par l'activité vitaminique. En outre, l'érythorbate de sodium possède un grade pharmaceutique et alimentaire.

Par ailleurs, l'association de polyéthylène glycol et d'érythorbate de sodium est particulièrement intéressante dans l'application considérée, et ce grâce à leurs actions protectrices complémentaires à l'égard des cellules et tissus en culture in vitro.

Cette action complémentaire vient du fait que l'érythorbate de sodium possède une action anti-oxydante sur le milieu biologique constituant l'environnement des tissus et cellules en culture, le polyéthylène glycol agissant principalement sur le maintien de l'intégrité structurelle et fonctionnelle desdits tissus et cellules.

Selon une autre réalisation la composition selon invention peut comprendre en outre une substance qui est complexée au polyéthylène glycol, par le procédé dit de « pégylation ». En effet, dans de nombreux domaines, le polyéthylène glycol est utilisé comme vecteur de molécules ayant une fonction thérapeutique, le polyéthylène glycol utilisé pouvant être de faible poids moléculaire (inférieur à 1000 g/mol) ou de poids moléculaire élevé (jusqu'à 100 000 g/mol).

Dans l'application considérée, cette substance complexée peut être un lipide, le polyéthylène glycol étant alors destiné à jouer le rôle de transporteur de lipides, de façon similaire à l'albumine dans les compositions contenant des protéines. Par exemple, du polyéthylène glycol de poids moléculaire égal à 900 daltons peut être fixé au cholestérol.

En outre, le polyéthylène glycol présente l'avantage de pouvoir se complexer à de nombreuses autres substances, ses possibilités de complexation étant plus étendues que celles de l'albumine. Par exemple, du polyéthylène glycol de poids moléculaire égal à 4500 ou 10000 daltons peut être fixé au facteur CSF granulocytaire humain, qui est un facteur de croissance spécifique des cellules souches hématopoiétiques, de sorte à augmenter l'activité de ce dernier au sein d'un milieu de culture cellulaire. En effet, il est connu que les facteurs de croissance au sein des milieux de culture présentent l'inconvénient de disparaître trop rapidement. Or, grâce à leur complexation avec du polyéthylène glycol, la stabilité des facteurs de croissance peut être améliorée.

L'invention a pour objet un milieu de culture comprenant une telle composition.

Ce milieu peut être obtenu par dissolution d'une composition telle que celle décrite ci-dessus, avec une concentration en polyéthylène glycol supérieure ou égale à 10 g/l.

Selon une réalisation, la concentration en polyéthylène glycol est comprise entre 10 et 50 g/l, et notamment entre 10 et 30 g/l.

En outre, un milieu de culture peut également être obtenu par dissolution d'une composition comprenant de l'érythorbate de sodium, la concentration en érythorbate de sodium étant inférieure ou égale à 1 g/l, et notamment comprise entre 0,01 mg/l et 1 g/l. Plus particulièrement, la concentration d'érythorbate de sodium est inférieure à 50 mg/l, et est notamment comprise entre 1 et 50 mg/l.

De tels milieux de culture peuvent être obtenus par dissolution, dans un volume donné de liquide, d'une composition préalablement formulée, c'est-à-dire par dissolution simultanée de l'ensemble des constituants d'une composition selon l'invention.

En variante, les milieux de culture peuvent être obtenus par dissolution séparée de certains constituants de la composition selon l'invention, de sorte à obtenir un milieu de culture dans lequel la concentration de chaque constituant correspond à la valeur requise.

On décrit à présent la structure générale d'un tel milieu de culture. Ce milieu comprend ainsi :
- une formule de base référencée, notamment l'IMDM (*Iscove's Modified Dulbecco's Medium*), le DMEM (*Dulbecco's Modified Eagle Medium*), le RPMI 1640 ou autres. Ces bases sont composées pour l'essentiel de sels inorganiques, d'acides aminés, de vitamines et d'autres composants, notamment le glucose pour son apport énergétique et l'HEPES pour son pouvoir tampon,
- des compléments de base tels que notamment des acides aminés non essentiels, des minéraux, des éléments traces,
- de l'insuline humaine recombinante ou un substitut constitué d'un sel de zinc,
- un chelateur de fer en tant que substitut de transferrine,
- du polyéthylène glycol (PEG), utilisé à une concentration inférieure à 50 g/l de milieu de culture et notamment 10, 20, 25 et 30 g/l,
- éventuellement de l'érythorbate de sodium, à une concentration comprise entre 0,01 mg/l et 1 g/l de milieu de culture et plus précisément aux concentrations comprises entre 1 et 50 mg/l,
- des compléments moléculaires spécifiques de la croissance et des activités métaboliques pour chaque type cellulaire cultivé.

Concernant ces compléments spécifiques, le milieu n'exclut pas l'utilisation de molécules d'origine végétale. Des lipides insolubles peuvent être également additionnés, sous une forme libre ou complexée, notamment avec des cyclodextrines.

Il est donc possible de procéder à des cultures de cellules animales ou de tissus en utilisant un milieu selon l'invention. A titre préliminaire, on décrit à présent quatre exemples de milieux de culture cellulaire qui sont optimisés pour deux applications distinctes. Les exemples 1 et 2 décrivent un milieu optimisé pour l'expansion des cellules souches et progéniteurs hématopoïétiques ; les exemples 3 et 4 définissent un milieu optimisé pour la culture d'hybridomes producteurs d'anticorps monoclonaux.

A ce jour, les milieux commerciaux les plus couramment utilisés pour la culture de cellules souches et progéniteurs hématopoïétiques appartiennent à la gamme X-VIVO. Selon les propres descriptions du fabriquant, ces milieux sont composés d'une formule de base, d'une albumine humaine de grade pharmaceutique, d'insuline humaine recombinante et de transferrine humaine pasteurisée. Le développement du milieu de culture a été conduit en comparaison avec ce standard de fabrication des milieux de culture sans sérum.

### Exemple 1 :

Un milieu d'expansion des cellules souches et progéniteurs hématopoïétiques a été défini selon la formule suivante:
- une base IMDM (*Iscove's Modified Dulbecco's Medium*) composée de : CaCl₂ anhydre (165 mg/L), KCl (330 mg/L), KNO₃ (0,076 mg/L), MgSO₄ anhydre (97,67 mg/L), NaCl (4505 mg/L), NaHCO₃ (3024 mg/L), NaH₂PO₄.H₂O (125 mg/L), Na₂SeO₃.5H₂O (0,01 mg/L), glucose (4500 mg/L), HEPES (5958 mg/L), rouge de phénol.Na (15 mg/L), pyruvate de sodium (110 mg/L), L-alanine (25 mg/L), L-arginine.HCl (84 mg/L), L-asparagine.H₂O (28,40 mg/L), acide L-aspartique (30 mg/L), L-cystine 2HCl (91,24 mg/L), acide L-glutamique (75 mg/L), L-glutamine (584 mg/L), glycine (30 mg/L), L-histidine.HCl.H2O (42 mg/L), L-isoleucine (105 mg/L), L-leucine (105 mg/L), L-lysine.HCl (146 mg/L), L-méthionine (30 mg/L), L-phénylalanine (66 mg/L), L-proline (40 mg/L), L-sérine (42 mg/L), L-thréonine (95 mg/L), L-tryptophane (16 mg/L), L-tyrosine.2Na.2H₂O (104,20 mg/L), L-valine (94 mg/L), D-biotine (0,013 mg/L), D- pantothénate de calcium (4 mg/L), chlorure de choline (4 mg/L), acide folique (4 mg/L), i-inositol (7,2 mg/L), nicotinamide (4 mg/L), pyridoxine.HCl (4 mg/L), riboflavine (0,4 mg/L), thiamine.HCl (4 mg/L), vitamines B₁₂ (0,013 mg/L),
- des compléments de base notamment des éléments traces,
- de l'insuline humaine recombinante (1-100 mg/L) ou un substitut d'insuline tel que le chlorure de zinc (0,1-10 mg/L),
- du gluconate de fer (II) (50-1000 mg/L),
- du PEG 20000 daltons (10-30 g/L),
- des compléments spécifiques pour l'expansion de ces cellules tels que : glutathion réduit (0,01-0,1 mg/L) et nucléosides (0,1-10 mg/L).

### Exemple 2 :

Un milieu d'expansion des cellules souches et progéniteurs hématopoïétiques, qui est identique à celui défini dans l'exemple 1, comprenant en outre de l'érythorbate de sodium (1-50 mg/L).

Des cellules hématopoïétiques primitives (CD34⁺) extraites de sang de cordon ombilical, ont été ensemencées à la concentration de 8000 cellules par ml dans un milieu de culture. Deux combinaisons de facteurs de croissance (cytokines) ont été additionnées pour orienter la croissance de ces cellules :
- le cocktail de cytokines n°1 est à base de SCF (50 ng/ml), TPO (50 ng/ml), FL (50 ng/ml), G-CSF (40 U/ml), GM-CSF (5 U/ml), IL-3 (1,7 U/ml) et IL-6 (10 U/ml),
- le cocktail de cytokines n°2 est à base de SCF (50 ng/ml), TPO (50 ng/ml), FL (50 ng/ml), IL-3 (1,7 U/ml) et IL-6 (10 U/ml).

Les cultures sont incubées 7 jours à 37°C dans une atmosphère saturée en humidité à 95% air / 5% CO₂. Après 7 jours d'expansion, la numération et la viabilité cellulaire ont été déterminées et les cellules ensemencées dans un milieu semi-solide (H4434, *StemCell Technologies*) afin de déterminer le taux d'expansion des progéniteurs clonogéniques. Les résultats sont exprimés dans le tableau 1.

**Tableau 1**

| **Milieu** | **Cytokines** | **Cellules à J0** | **Cellules à J7** | **Viabilité** | **Expansion** |
|---|---|---|---|---|---|
| Base IMDM | Cocktail n° 1 | 8000 | 505000 | 88,7% | 63 x |
| ME2^{*} sans PEG 3% | Cocktail n° 1 | 8000 | 1185000 | 85,3% | 148 x |
| ME2 avec PEG 3% | Cocktail n°1 | 8000 | 1755000 | 93,8% | 219 x |
| X-VIVO 15 | Cocktail n°1 | 8000 | 18000000 | 91,8% | 225 x |
| ME2 avec PEG 3% | Cocktail n°2 | 8000 | 907000 | 97,5% | 113 x |
| X-VIVO 15 | Cocktail n°2 | 8000 | 1063000 | 93,7% | 132 x |

| | | | | | |
|---|---|---|---|---|---|
| * ME2 = Milieu Selon l'exemple 2 | | | | | |

Ces résultats montrent que le cocktail de cytokines n°1 induit une expansion limitée des cellules (63x) dans la base IMDM utilisée pour la préparation du milieu selon l'invention. L'addition des compléments à l'exception du PEG améliore les résultats d'expansion (148x). Enfin, l'addition de PEG renforce les compétences du milieu selon l'invention et permet d'obtenir un taux d'expansion (219x) comparable au milieu de référence (225x).

Cette similitude de performances est confirmée avec l'utilisation du cocktail de cytokines n°2. Dans tous les cas, la viabilité cellulaire mesurée après 7 jours de culture, est excellente (de 85 à 97%). Les cultures clonogéniques ont alors démontré la qualité du milieu de culture selon l'exemple 2 en terme d'amplification des progéniteurs clonogéniques (progéniteurs capables de donner une colonie de cellules *in vitro* dans un milieu semi-solide).

L'analyse des colonies obtenues est détaillée dans le tableau 2.

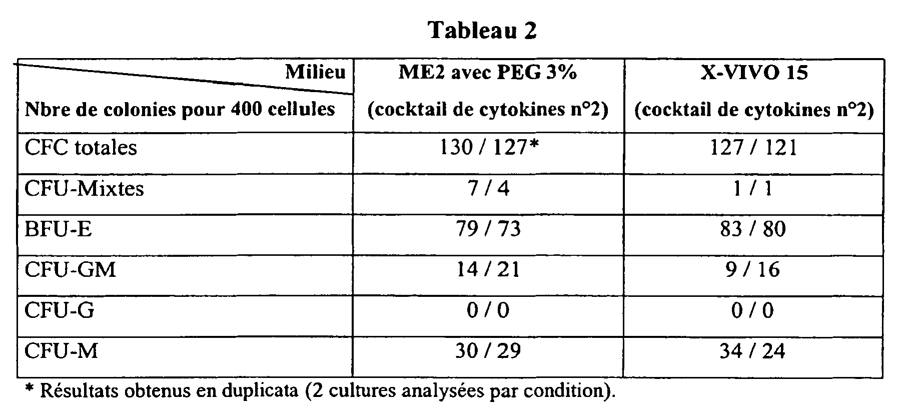

L'indice de clonogénicité (nombre de colonies totales comptées pour 100 cellules ensemencées) est invariant entre le milieu selon l'invention et son témoin (de l'ordre de 130 colonies comptées pour 400 cellules testées). La répartition entre les différents types de colonies est également similaire avec toutefois un avantage pour le milieu selon l'invention : les progéniteurs d'intérêts CFU-Mixtes et CFU-GM sont mieux représentés. L'impact du PEG sur le taux d'expansion des cellules humaines CD34+ de sang de cordon et sur la conservation particulière des progéniteurs clonogéniques du type CFU-Mixtes a pu être analysé dans d'autres formulations du milieu selon l'invention ainsi que dans des milieux commerciaux de référence (résultats non montrés).

### Exemple 3:

Un milieu pour la culture industrielle d'hybridomes producteurs d'anticorps monoclonaux a été défini selon la formulation suivante :
- une base RPMI 1640 composée de : Ca(NO₃)₂.4H₂O (100 mg/L), KCl (400 mg/L), MgSO₄.7H₂O (100 mg/L), NaCl (5000 mg/L), NaHCO₃ (2000 mg/L), Na₂HPO₄.7H₂O (1512 mg/L), glucose (2000 mg/L), glutathione (reduced) (1 mg/L), HEPES (5957,4 mg/L), rouge de phénol.Na (5 mg/L), L-arginine (200 mg/L), L-asparagine H₂O (50 mg/L), acide L-aspartique (20 mg/L), L-cystine (50 mg/L), acide L-glutamique (20 mg/L), L-glutamine (300 mg/L), glycine (10 mg/L), L-Histidine (15 mg/L), hydroxy-L proline (20 mg/L), L-isoleucine (50 mg/L), L-leucine (50 mg/L), L-lysine.HCl (40 mg/L), L-méthionine (15 mg/L), L-phénylalanine (15 mg/L), L-proline (20 mg/L), L-sérine (30 mg/L), L-thréonine (20 mg/L), L-tryptophane(5 mg/L), L-tyrosine (20 mg/L), L-valine (20 mg/L), acide p-aminobenzoïque (1 mg/L), D-biotine (0,2 mg/L), D-pantothénaté de calcium (0,25 mg/L), chlorure de choline (3 mg/L), acide tolique (1 mg/L), i-inositol (35 mg/L), nicotinamide (1 mg/L), pyridoxine.HCl (1 mg/L), riboflavine (0,2 mg/L), thiamine.HCl (1 mg/L), vitamine B₁₂ (0,005 mg/L),
- des compléments de base notamment des éléments traces,
- un substitut d'insuline tel que le chlorure de zinc (0,1-10 mg/L),
- du gluconate de fer (II) (50-1000 mg/L),
- du PEG 20000 daltons (10-30 g/L),
- des compléments spécifiques pour l'expansion des hybridomes tels que : nucléosides (0,1-10 mg/L), pyruvate de sodium (1-100 mg/L), putrescine. 2HCl (0,05-5 mg/L), hypoxanthine (0,1-10 mg/L).

### Exemple 4 :

Un milieu pour la culture industrielle d'hybridomes producteurs d'anticorps monoclonaux a été défini de façon identique à celle de l'exemple 3, avec en outre de l'erythorbate de sodium (1-50 mg/L).

Pour la culture d'hybridomes producteurs d'anticorps monoclonaux, les bases IMDM et DMEM/HAM-F12 (1/1) sont également recommandées. Les compléments du milieu selon l'invention sont alors définis en fonction de la base utilisée : ainsi, la base DMEM/HAM-F12 intègre directement des molécules telles que la putrescine, l'hyoxanthine, la thymidine ou bien encore du pyruvate de sodium.

Le milieu de culture obtenu à partir d'une composition peut donc être utilisé pour la culture de cellules animales et de tissus à visée thérapeutique, notamment pour :
- la culture de cellules souches somatiques, notamment les cellules souches et progéniteurs hématopoïétiques issus de la moelle osseuse, du sang périphérique ou du sang de cordon ombilical,
- la culture et l'activation de cellules immunitaires, notamment les lymphocytes tels que : PBL (*peripheral blood lymphocytes*) ; LAK (*lymphokine activated killer cells*) ; TIL (*tumor infiltrating lymphocytes*),
- la culture des monocytes et macrophages,
- la production de cellules présentatrices d'antigènes, notamment les cellules dendritiques,
- la culture des hépatocytes, notamment pour la transplantation ou la préparation d'un foie bioartificiel,
- la culture des îlots de Langerhans, notamment dans le cadre du traitement des diabètes,
- l'expansion et la différentiation des cellules souches mésenchymateuses, notamment dans le cadre de la réparation des déficits osseux,
- la culture de cellules musculaires, notamment dans le cadre du traitement des déficiences cardiaques,
- la culture des cellules neuronales, somatiques, foetales ou embryonnaires, notamment dans le cadre du traitement des maladies neurodégénératives,
- la culture des cornées ou autres tissus,
- la culture de cellules souches embryonnaires,
- la décongélation, le lavage et la congélation des cellules et tissus à visée thérapeutique.

Suivant l'invention, par exemple pour certaines préparations de cellules humaines à visée thérapeutique, l'albumine est substituée partiellement par le PEG. A cet effet, le milieu de culture est obtenu d'une part par dissolution de la composition suivant l'invention et d'autre part par ajout de la quantité souhaitée d'albumine. Par exemple, la quantité d'albumine ajoutée dans le milieu de culture peut être inférieure à 5 g/l, c'est-à-dire qu'on substitue typiquement jusqu'à 50 % de l'albumine nécessaire par du PEG.

Pour résoudre les problèmes de contaminations potentielles liées à l'utilisation de protéines animales, il est préférable d'utiliser alors de l'albumine humaine injectable (HSA).

Par exemple, l'albumine joue un rôle prépondérant dans l'expansion des cellules souches hématopoïétiques issues de sang périphérique normal (cellules difficiles à amplifier), elle peut également être utile à la récupération des cellules dendritiques (contribution au décollement des cellules de leur support plastique).

On donne ci-dessous un exemple de réalisation d'un milieu de culture contenant de l'albumine.

### Exemple 5 :

Des cellules hématopoïétiques primitives (CD34⁺) extraites de sang de cordon ombilical, ont été ensemencées à la concentration de 8000 cellules par ml dans un milieu de culture. Des facteurs de croissance (cytokines) ont été additionnés pour orienter la croissance de ces cellules : SCF (40 ng/ml), TPO (40 ng/ml), FL (40 ng/ml), IL-3 (1,7 U/ml) et IL-6 (10 U/ml). Les cultures sont incubées 7 jours à 37°C dans une atmosphère saturée en humidité à 95% air/5% CO₂. Après 7 jours d'expansion, la numération et la viabilité cellulaire ont été déterminées. Les résultats sont détaillés dans le tableau 3.

**Tableau 3**

| **Milieu** | **Cellules** à **J0** | **Cellules à J7** | **Viabilité** | **Expansion** |
|---|---|---|---|---|
| Composition sans PEG + 1% HSA | 8000 | 2436840 | 93,3% | 304 x |
| Composition sans PEG + 0,3% HSA | 8000 | 1979600 | 89,4% | 247 x |
| Composition sans PEG + 0,2% HSA | 8000 | 1940400 | 88,4% | 242 x |
| Composition avec PEG 1,5% + 0,3% HSA | 8000 | 2391200 | 93,1% | 299 x |
| Composition avec PEG 1,5% + 0,2% HSA | 8000 | 2152840 | 92,2% | 269 x |

Ces résultats montrent que le taux d'expansion de ces cellules diminue lorsque la teneur en albumine diminue (de 304x à 242x pour un taux en albumine allant de 10 g/l à 2 g/l). La substitution partielle de l'albumine, notamment par le PEG 20000 daltons, permet de maintenir les performances du milieu de culture (exemple : 299x avec un teneur en albumine limitée à 3 g/l).

Ainsi, les propriétés semblables et additives de l'albumine et de son substitut permettent d'envisager une réduction significativement du taux d'albumine et par conséquent, du coût d'exploitation de tels milieux.

Compte tenu de ses propriétés et de son faible coût d'exploitation, le milieu de culture obtenu à partir d'une composition selon l'invention s'ouvre également à un vaste champ d'applications dans le domaine industriel biotechnologique comprenant notamment :
- la culture d'hybridomes producteurs d'anticorps monoclonaux, notamment lorsque ces anticorps sont directement injectables chez l'homme ; dans cette application, le milieu selon l'invention présente l'intérêt d'être dépourvu de toute immunoglobuline contaminante.
- la préparation de molécules produites par culture de cellules, notamment les nombreuses lignées transfectées ou non de myélomes et CHO (*Chinese Hamster Ovary cells*), à des fins thérapeutique, vaccinale, diagnostique ou de recherche.
- la préparation d'entités biologiques par culture de cellules, notamment la production de vecteurs viraux au service des thérapies géniques.

## Revendications

1. Milieu de culture pour la culture de cellules animales ou de tissus, comprenant un substitut de transferrine naturelle, un substitut d'insuline naturelle, du polyéthylène glycol dans une concentration supérieure ou égale à 10 g/l, et de l'albumine.

2. Milieu de culture selon la revendication 1, **caractérisé en ce qu'**il comprend en outre de l'érythorbate de sodium.

3. Milieu de culture selon la revendication 2, **caractérisé en ce que** la concentration en érythorbate de sodium est inférieure ou égale à 1 g/l, notamment inférieure à 50 mg/l.

4. Milieu de culture selon l'une des revendications 1 à 3, **caractérisé en ce que** la concentration du polyéthylène glycol est comprise entre 10 et 50 g/l, notamment entre 10 et 30 g/l.

5. Milieu de culture selon l'une des revendications 1 à 4, **caractérisé en ce que** le polyéthylène glycol est de poids moléculaire compris entre 50 et 100 000 daltons, notamment 20 000 daltons.

6. Milieu de culture selon l'une des revendications 1 à 5, **caractérisé en ce qu'**il comprend en outre une substance qui est complexée au polyéthylène glycol.

7. Milieu de culture selon la revendication 6, **caractérisé en ce que** la substance complexée est un lipide.

8. Milieu de culture selon la revendication 6, **caractérisé en ce que** la substance complexée est un facteur de croissance.

9. Milieu de culture selon l'une des revendications 1 à 8, **caractérisé en ce que** la concentration en albumine est inférieure ou égale à 5 g/l.

## Claims

1. Culture medium for the culture of animal cells or tissue, comprising a natural transferrin substitute, a natural insulin substitute, polyethylene glycol in a concentration equal to or greater than 10 g/l, and albumin.

2. Culture medium according to claim 1, **characterised in that** is also contains sodium erythorbate.

3. Culture medium according to claim 2, **characterised in that** the sodium erythorbate concentration does not exceed 1 i g/l, in particular is under 50 mg/l.

4. Culture medium according to any of claims 1 to 3, **characterised in that** the concentration of the polyethylene glycol is between 10 and 50 g/l, in particular between 10 and 30 g/l.

5. Culture medium according to any of claims 1 to 4, **characterised in that** the molecular weight of the polyethylene glycol is between 50 and 100,000 daltons, in particular 20,000 daltons.

6. Culture medium according to any of claims 1 to 5, **characterised in that** it also comprises a substance that is complexed with polyethylene glycol.

7. Culture medium according to claim 6, **characterised in that** the complexed substance is a lipid.

8. Culture medium according to claim 6, **characterised in that** the complexed substance is a growth factor.

9. Culture medium according to any of claims 1 to 8, **characterised in that** the albumin concentration does not exceed 5 g/l.

## Patentansprüche

1. Kulturmilieu für die Kultur von tierischen Zellen oder Geweben mit einem Substitut aus natürlichem Transferrin, einem Substitut aus natürlichem Insulin, Polyethylenglykol in einer Konzentration von größer als oder gleich 10 g/l und Albumin.

2. Kulturmilieu gemäß Anspruch 1, **dadurch gekennzeichnet, dass** es darüber hinaus Natriumerythorbat enthält.

3. Kulturmilieu gemäß Anspruch 2, **dadurch gekennzeichnet, dass** die Konzentration aus Natriumerythorbat geringer als oder gleich 1 g/l, insbesondere geringer als 50 mg/ 1 ist.

4. Kulturmilieu gemäß Anspruch 1 bis 3, **dadurch gekennzeichnet, dass** die Konzentration des Polyethylenglykols zwischen 10 und 50 g/l, insbesondere zwischen 10 und 30 g/l, inbegriffen ist.

5. Kulturmilieu gemäß Anspruch 1 bis 4, **dadurch gekennzeichnet, dass** das Polyethylenglykol ein zwischen 50 und 100.000 Dalton, insbesondere 20.000 Dalton inbegriffenes Molekulargewicht aufweist.

6. Kulturmilieu gemäß Anspruch 1 bis 5, **dadurch gekennzeichnet, dass** es darüber hinaus eine Substanz umfasst, die in Polyethylenglykol komplexiert ist.

7. Kulturmilieu gemäß Anspruch 6, **dadurch gekennzeichnet, dass** die komplexierte Substanz ein Lipid ist.

8. Kulturmilieu gemäß Anspruch 6, **dadurch gekennzeichnet, dass** die komplexierte Substanz ein Wachstumsfaktor ist.

9. Kulturmilieu gemäß Anspruch 1 bis 8, **dadurch gekennzeichnet**, das die Konzentration an Albumin geringer als oder gleich 5 g/l ist.
